**Europäisches Patentamt**

**European Patent Office**

**Office européen des brevets**

(19)

(11) Publication number : **0 489 104 B1**

(12)

# EUROPEAN PATENT SPECIFICATION

(45) Date of publication of patent specification :
**15.12.93 Bulletin 93/50**

(51) Int. Cl.[5] : **D21C 9/10, C12S 3/08**

(21) Application number : **90913469.4**

(22) Date of filing : **24.08.90**

(86) International application number :
**PCT/DK90/00220**

(87) International publication number :
**WO 91/02839 07.03.91 Gazette 91/06**

(54) PROCESS FOR TREATMENT OF LIGNOCELLULOSIC PULP.

(30) Priority : **25.08.89 DK 4202/89**

(43) Date of publication of application :
**10.06.92 Bulletin 92/24**

(45) Publication of the grant of the patent :
**15.12.93 Bulletin 93/50**

(84) Designated Contracting States :
**AT BE CH DE DK ES FR GB IT LI LU NL SE**

(56) References cited :
**WO-A-89/08738**
**Viikari L. et al, "Fourth International Symposium on Wood and Pulping Chemistry", Paris (1987), p. 151-154, see especially page 154, column 1, line 11 - column 2, line 25**
**Chauvet J.-M. et al, "Fourth International Symposium on Wood and Pulping Chemistry", Paris (1987), p. 325-327, see especially page 326, column 1, line 27 - column 2, line 16.**

(56) References cited :
**Biotechnology and Bioengineering, Vol. 32, July 1988 M.G. Paice et al: "Viscosity-Enhancing Bleaching of Hardwood Kraft Pulp with Xylanase from a Cloned Gene", see page 235-page 239 especially page 237, column 1, line 1-line 20**

(73) Proprietor : **NOVO NORDISK A/S**
**Novo Allé**
**DK-2880 Bagsvaerd (DK)**

(72) Inventor : **PEDERSEN, Lars, Saaby**
**Bagsvaerdvej 69B.14**
**DK-2800 Lyngby (DK)**
Inventor : **SKJOLD-JOERGENSEN, Steen**
**Georginevej 12**
**DK-2970 Hoersholm (DK)**
Inventor : **ANKER, Lisbeth**
**Athensvej 16**
**DK-2300 Koebenhavn S (DK)**
Inventor : **AUSTIN, Helen, F.**
**31 Greenbriar Lane**
**Newtown, CT 06470 (US)**

(74) Representative : **Bach, Niels et al**
**c/o Novo Nordisk A/S Patent Dept. Novo Allé**
**DK-2880 Bagsvaerd (DK)**

## Description

A common stage in the treatment of wood chips for paper production is chemical pulping, e.g. the so-called Kraft process which is an alkaline sulphate cooking of the wood chips. The native wood chips contain around 30% lignin, and at the end of the chemical cooking process less than 5% of the lignin compounds is still left in the pulp. Due to the strong brown color of the remaining lignin and its tendency to darken in UV light or by oxidation this has to be removed in order to obtain a white pulp without the tendency to color reversion.

The brown color can be removed by a multistage bleaching using e.g. chlorine and/or chlorine dioxide. However, due to the ever increasing environmental concern, the dosage of chlorine and/or chlorine dioxide has to be kept at a minimum, and for that reason the enzymatic treatment of the Kraft cooked pulp has been introduced, vide e.g. The third International Conference on Biotechnology in the Pulp and Paper Industry, Stockholm, 16-19.6, 1986, page 67-69.

Hitherto the enzymatic treatment has been carried out at acid pH with hemicellulases with a pH optimum in the acid pH-region, vide 4th International Symposium Wood and Pulping Chemistry, Paris, 22-30 April, 1987, Vol. 1, page 151-154.

The fact that the enzymatic treatment hitherto has to be carried out at an acid pH is a disadvantage, because the alkaline Kraft pulp has to be acidified, which requires big amounts of chemicals, and also, it disturbs the salt balance, which can be a problem in the recovery system of the pulp mill.

For certain chemical pulping processes, e.g. Kraft pulping, it would be highly advantageous if the enzymatic treatment could be carried out at alkaline pH, thus avoiding the need for neutralization of the pulp, and making the enzyme process directly adaptable to the existing process hardware.

Surprisingly, according to the invention it has been found that alkaline xylanases exhibiting an excellent bleachability improving effect actually exist. Furthermore, it has been found that the time period necessary for enzymatic pretreatment can be shortened considerably with such alkaline xylanases, and also, use of the alkaline xylanases is accompanied by a reduced need for active chlorine in the bleaching stages compared to the acid treatments described in the literature and/or the brightness is improved.

Thus, the process for treatment of lignocellulosic chemical pulp is characterized by the fact that the lignocellulosic pulp is treated with an alkaline xylanase at a pH above 7.5, whereafter the thus treated lignocellulosic pulp is treated with chlorine at an active chlorine multiple of 0.20 or less in the first chlorination stage.

In Biotechnology and Bioengineering, Vol. 32, pages 235-239 (1988) M.G. Paice et al. describe a process for treatment of lignocellulosic chemical pulp. However, it appears from page 236, second column, line 7 above the lignin formula, that the enzyme treatment was carried out at pH 5.0, i.e. clearly outside the scope of the invention.

In Fourth International Symposium on Wood and Pulping Chemistry, Paris (1987) Jean-Marie Chauvet et al. describe a process for treatment of lignocellulosic pulp at an acid pH with hemicellulases with a pH-optimum in the acid pH-region, i.e. a prior art process.

In this specification with claims an alkaline xylanase is a hemicellulase which exhibits a high xylanase activity at high pH values, as specified in more detail in the following.

In this specification with claims an alkaline xylanase is defined as a hemicellulase which at a pH of 9.0 retains at least 10% of its maximum xylanase activity (activity at optimum pH). The xylanase activity at a given pH is measured as the Kappa no. reduction obtained in the experimental procedure described below.

Experimental conditions: The substrate is a well washed Birch Kraft pulp having a Kappa no. of 15 to 25 and a viscosity of 1200-1500 $dm^3$/kg (viscosity measured in cupri ethylene diamine solution according to the SCAN standard SCAN-CM 15:88).

Buffer solutions: In the pH range from 5.8 to 8.0 a 0.05 M potassium dihydrogen phosphate buffer is used, and in the pH range from 8.0 to 10.8 a 0.0125 M borate buffer is used.

Procedure: A sample of pulp is disintegrated according to the Scan standard (SCAN-C18:65). A buffer solution having the desired pH is used in the pulper instead of water.

After pulping the pulp is drained on a wire screen to a concentration of approximately 25%. The pulp is then diluted to 10% dry substance using a buffer solution of the desired pH, which contains the xylanase in solution. 500 EXU-units of xylanase is added per kg of dry pulp. EXU is an abbreviation for endoxylanase activity units, to be defined later.

The pulp and xylanase is mixed thoroughly and placed on a water bath at 50°C for 3 hours.

After the 3 hours of enzyme treatment the pulp is washed with water on a wire screen and the Kappa no. of the well-washed pulp is determined.

The above described procedure is repeated as a control experiment, i.e. without addition of xylanase.

At a given pH the reduction in Kappa no. is calculated as (Kappa no. of control minus Kappa no. of xylanase treated pulp).

If less xylanase than 10 EXU/kg of dry pulp is used, no significant improvement of the bleacheability of the pulp can be detected.

The alkaline hemicellulase preparations usually contain cellulase activity as a side activity. In order to avoid decomposition of the cellulose to any undue extent, the cellulase activity should be small in comparison to the xylanase activity. Thus, an enzyme dosage giving rise to a cellulase activity of more than 1000 EGU units/kg of dry pulp (the EGU unit for cellulase activity to be defined later) should preferably not be used. The EXU activity unit for xylanase activity is defined in AF-293.9/1, which can be obtained on request from Novo Nordisk a/s, Novo Allé, DK-2880 Bagsvaerd, Denmark. The EGU activity unit for cellulase activity is defined in AF-275/1, which can be obtained on request from Novo Nordisk a/s, Novo Allé, DK-2880 Bagsvaerd, Denmark.

As indicated, the treatment with the alkaline xylanase precedes the chlorine treatment; however, the chlorine treatment does not necessarily follow directly after the treatment with the alkaline xylanase.

The chlorine multiple is the factor which multiplied by the Kappa no. of the pulp gives the dosage of active chlorine in the first bleaching stage as % (w/w) on dry pulp.

The Kappa no. is a measure of the lignin content and is defined in SCAN-C 1:77 (Scandinavian Pulp, Paper and Board Testing Committee).

Due to the fact that the enzyme preparations used according to the invention are hemicellulase complexes, these preparations usually also contain other polyose degrading enzymes as side activities to the xylanase activity, e.g. galactomannases and arabinosidases. The xylanase activity is the key activity, but other hemicellulase activities are also desirable for obtaining the bleacheability improving effect.

Thus, the process for treatment of lignocellulosic Kraft pulp is a new combination of two items: 1) the use of alkaline xylanases, and 2) the use of a low multiple, lower than the commonly used multiple in traditional bleaching processes in the range of 0.20-0.25.

Summing up, the process for bleaching of enzymatically treated lignocellulosic pulp according to the invention is accompanied by the following advantages:

1) there is no need for neutralization of the alkaline pulps, e.g. Kraft brown stock.

2) The time for enzyme treatment can be shortened considerably.

3) The low multiple means that the chlorine addition is reduced, which in itself is accompanied by a number of advantages (economic, easier treatment of waste water, smaller amounts of toxic substances).

4) Higher brightness.

In a preferred embodiment of the process according to the invention the xylanase treatment is performed at temperatures between 40 and 100°C, preferably between 40 and 80°C, more preferably between 50 and 70°C. The temperature interval of 40-100°C is the normal pulp temperature at this stage, and it has been found that the activity and stability of the alkaline xylanases used in the invention is satisfactory in this interval.

In a preferred embodiment of the process according to the invention the xylanase treatment is performed over a period of 15 minutes to 24 hours, preferably between 30 minutes and 5 hours, most preferably between 30 minutes and 3 hours. With reasonable xylanase activities compatible with a sound commercial economy it has been found that the xylanase has performed its hydrolysing activity to the desired degree of hydrolysis in this short period of time, whereas 12-24 hours is the usual prior art time period for the xylanase treatment, vide Viikari, L. et al., Third International Conference on Biotechnology in the Pulp and Paper Industry, Stockholm 16-19.6.1986.

In a preferred embodiment of the process according to the invention the xylanase treatment takes place at a consistency of 5-35%, preferably 8-25%, most preferably 8-15%. The consistency is the dry matter content of the pulp. A pulp with a consistency above 35% is difficult to mix effectively with the enzyme preparation, and a pulp with a consistency below 5% carries too much water, which is a disadvantage from an economic point of view.

In a preferred embodiment of the process according to the invention the xylanase is producible by means of the microorganisms Malbranchea cinnamomea, Humicola insolens, Bacillus pumilus, Bacillus stearothermophilus, Thermonmonospora fusca or Streptomyces olivochromogenes. These microorganisms are able to produce alkaline xylanases, which can be used in the process according to the invention. Other microorganisms which are able to produce alkaline xylanases, can be located by means of the routine test described earlier in this specification.

In a preferred embodiment of the process according to the invention an (EOP) treatment is introduced between the xylanase treatment and the chlorine treatment. An (EOP) stage is an (EO) extraction stage reinforced with hydrogen peroxide, as described by e.g. Helming, O. et al., Tappi Journal, Vol 72, No. 7 (1989), p. 55-61. By means of this supplementary process the Kappa no. of the enzyme treated pulp is lowered considerably more than what is normally the case after an (EOP) stage.

In a preferred embodiment of the process according to the invention the chlorine multiple is between 0.10 and 0.20. Even a small reduction in chlorine multiple is a significant improvement, as a small reduction in the amount of chlorine used significantly cuts back on the formation of chlorinated organic compounds, e.g. chlorinated dioxins, vide Kringstad, K. Sv. Papperstidning, Vol. 92, no. 6, 1989, p. 42-44.

In order to illustrate the process according to the invention reference will be made to the following examples.

The bleaching stages are abbreviated as the C, D, and E processes, the C process being the chlorine bleaching stage, the D process being the chlorine dioxide bleaching stage, and the E process being the alkaline extraction stage, vide The Bleaching of Pulp, Rudra P. Singh. Tappi Press, 1979.

The ISO-brightness is defined in International Standard, ISO, 2470/1977(E), Paper and Board - Measurement of diffused blue reflectance factor (ISO Brightness).

**EXAMPLE 1**

Production of Malbranchea cinnamomea xylanase

Malbranchea cinnamomea (syn. M. sulfurea, M. pulchella var. sulfurea) culture UAMH 2485 (UAMH indicates University of Alberta Mold Herbarium culture collection, Arberta, Canada) was maintained on YPG agar slants at 45°C.

Composition of YPG-agar:

| | |
|---|---|
| Glucose | 15 g/l |
| Yeast extract (from Difco) | 4 g/l |
| $K_2HPO_4$ | 1 g/l |
| $MgSO_4, 7H_2O$ | 0.5 g/l |
| Agar | 20 g/l |

Autoclaved at 121°C for 20 minutes

120 shake flasks with 150 ml XYH-4 medium each inoculated from YPG-agar slants and cultivated (at 250 rpm. with appr. 2 cm amplitude) at 45°C for 6 days.

Composition of XYH-4:

| | |
|---|---|
| Yeast Extract (from Difco) | 5 g/l |
| $KH_2PO_4$ | 5 g/l |
| Xylose | 10 g/l |
| Pluronic 61 | 0.1 ml/l |

pH is adjusted to 6 (by NaOH/ $H_2SO_4$)
Autoclaved at 121°C for 40 minutes

The mycelium was removed from the broth by filtration through a 100 μm nylon filter and a 10 μm nylon filter. The filtrate (a total of 9,2 l) was concentrated by ultrafiltration (and washed 3 times with one volume water) to 500 ml by a Pellicon ultrafiltration apparatus from Millipore with a 10,000 MW cut-off filter sheet). The resulting concentrate was freeze dried, whereby 6,25 g of powder was generated.

**EXAMPLE 2**

Production of Bacillus pumilus xylanase

Bacillus pumilus culture DSM 6124 (deposited on July 23, 1990 at Deutsche Sammlung von Mikroorganismen under the conditions of the Budapest Treaty) was maintained on A3-medium, at 37°C.

**A3-medium:**

|  | g/l |
|---|---|
| Peptone, Difco | 6.0 |
| Yeast Extract | 3.0 |
| Pepticase | 4.0 |
| Beef Extract | 1.5 |
| Glucose | 1.0 |
| Agar, Merck | 20.0 |
| $H_2O$ | 1000 ml |

pH adjustment to 7,3 before autoclaving.
Autoclaving 25 min./121°C.

120 shake flasks with 150 ml XYL-8 medium each inoculated from A3-agar slants were cultivated for 4 days, 37°C, 250 rpm with appr. 2 cm amplitude.

**XYL-8 medium:**

|  | g/l |
|---|---|
| Bacto-peptone Difco | 10.0 |
| Yeast extract | 10.0 |
| $K_2HPO_4$ | 15.0 |
| $MgSO_4 . 7H_2O$ | 0.5 |
| KCl | 0.1 |
| $FeSO_4 . 7H_2O$ | 0.01 |
| Beech xylan Lenzing | 6.0 |

pH adjustment to 7.0 before autoclaving.
Autoclaving 25 min./121°C.

The broth was centrifuged for half an hour at 4.000 rpm (SORVALL RC-3B centrifuge with a 6000 A rotor) The supernatant, 7.3 l, was filtered through a 10 μm nylonfilter, and concentrated by ultrafiltration by means of a Pellicon equipment from Millipore with a 10.000 MW cut-off membrane and washed 2 times with one volume of water. This resulted in 540 ml of concentrate. The concentrate was then lyophilized, whereby 8.8 g of powder was generated.

## EXAMPLE 3

Production of Bacillus stearothermophilus xylanase.

Use was made of Bacillus stearothermophilus culture NR. B-18659 (Agricultural Research, Culture Collection, Peoria, Illinois, USA).

Fed batch fermentation with the above Bacillus stearothermophilus culture was performed by means of a batch medium containing xylan along with a 50% (w/w%) xylose feed. The pH was controlled not to drop below pH 6.5 by means of 4N $NH_4OH$. The following ingredients were used:

Beech xylan [Linzing]     5.0 g/l
Corn steep liquor     10.0 g/l
yeast extract     2.0 g/l
$[NH_4]_2SO_4$     2.0 g/l
$K_2HPO_4$     2.0 g/l
$KH_2PO_4$     0.5 g/l

5

| | |
|---|---|
| CaCl$_2$ 2H$_2$O | 0.5 g/l |
| MgSO$_4$ 7H$_2$O | 0.5 g/l |
| Trace metals AEF-4 | 0.15 ml/l |

The feed was started at 22 hours at a constant rate of 3.3 ml/l/hr. The feed was stopped at 90 hours and the fermentation was stopped at 95 hours. The enzyme preparation is a liquid preparatopm obtained after ultrafiltration with 10,000 molecular weight cut-off (MWCO) filter- and stabilization with 20% sorbitol.

**EXAMPLE 4**

Production of Humicola insolens xylanase.

Humicola insolens culture ATCC 22082 (ATCC indicates American Type Culture Collection, Rockville, MD, USA) was maintained on YPG agar slants at 37°C.

Composition of YPG-agar:

| | |
|---|---|
| Glucose | 15 g/l |
| Yeast extract (from Difco) | 4 g/l |
| K$_2$HPO$_4$ | 1 g/l |
| MgSO$_4$, 7H$_2$O | 0.5 g/l |
| Agar | 20 g/l |

Autoclaved at 121°C for 20 minutes

100 shake flasks with 150 ml XYH-9 medium each inoculated from YPG-agar slants and cultivated (at 250 rpm. with appr. 2 cm amplitude) at 37°C for 5 days.

Composition of XYH-9:

| | |
|---|---|
| Wheat bran | 50 g/l |
| Corn steep liqueur | 30 g/l |
| NaH$_2$PO$_4$ | 5 g/l |

pH is adjusted to 6 (by NaOH/H$_2$SO$_4$)
Autoclaved at 121°C for 40 minutes

The broth was centrifuged for 35 minutes at 4000 rpm by means of a Sorval RC-3B centrifuge with a 6000 A rotor. The supernatant (a total of 5,4 l) was filtered through a nylon filter (10 μm) and concentrated by ultrafiltration (and washed 3 times with one volume water) to 760 ml by a Pellicon ultrafiltration apparatus from Millipore with a 10,000 MW cut-off filter sheet). The resulting concentrate was freeze dried, whereby 19,1 g of powder was generated.

**EXAMPLE 5**

Production of Thermonmonospora fusca xylanase

Thermonmonospora fusca ATCC 27730 (American Type culture Collection) was maintained on A3-medium at 37°C.

6

```
A3 medium:
                                                          g/l
          Peptone, Difco                                  6.0
          Yeast Extract                                   3.0
          Pepticase                                       4.0
          Beef Extract                                    1.5
          Glucose                                         1.0
          Agar, Merck                                    20.0
          H₂O                                       1000 ml
```

pH adjustment to 7.3 before autoclaving.
Autoclaving 25 min./121°C.

Thermonmonospora fusca was grown on A3-slants for 5 days, 37°C. The culture was resuspended in sterile water and inoculated in shake flasks with medium XYL-10a. Shake flasks of 100 ml were grown for 2 days, 45°C, 250 rpm, amplitude appr. 2 cm.

```
XYL-10a medium:
                                          g/l
          Soya-grit                      20.0
          Na-caseinat                    10.0
          Na₂HPO₄                         9.0
          Beech xylan Lenzing             3.0
          Pluronic 61 L                 0.1 ml
          H₂O                         1000 ml
```

pH adjustment to 7.5 before autoclaving.
Autoclaving 30 minutes/121°C.

Hereafter a 3 l Applicon fermentor, filled to 2.5 l with XYL-10a substrate was incubated with 5% of the fermentation volume. The fermenter was run for 4 days, 45°C, 700 rpm, 2 NL/min. The culture broth was centrifuged for 30 min. at 4000 rpm Sorwall RC-3B with a 6000 A rotor, and hereafter filtered through a 10 μm filter. The resulting volume of 2470 ml was then ultrafiltered through a 10.000 MW cut-off membrane using Pellicon ultrafiltration equipment from Millipore. Result 275 g of liquid.

**EXAMPLE 6**

Production of Streptomyces olivochromogenes xylanase

Streptomyces olivochromogenes DSM 6126 (deposited on July 23, 1990 at Deutsche Sammlung von Mikroorganismen under the conditions of the Budapest Treaty) was maintained on A3-medium at 30°C.

A3 medium:

|  | g/l |
|---|---|
| Peptone, Difco | 6.0 |
| Yeast Extract | 3.0 |
| Pepticase | 4.0 |
| Beef Extract | 1.5 |
| Glucose | 1.0 |
| Agar, Merck | 20.0 |
| $H_2O$ | 1000 ml |

pH adjustment to 7.3 before autoclaving.
Autoclaving 25 min./121°C.

Streptomyces olivochromogenes was grown on A3-slants for 5 days, 30°C. The culture was resuspended in sterile water and inoculated in shakeflask with medium XYL-10. Shake flasks of 100 ml were grown for 2 days, 30°C, 250 rpm, appr. amplitude 2 cm.

XYL-10 medium:

|  | g/l |
|---|---|
| Soya-grit | 20.0 |
| Na-caseinat | 10.0 |
| $Na_2HPO_4$ | 9.0 |
| Beech xylan Lenzing | 3.0 |
| Pluronic 61 L | 0.1 ml |
| $H_2O$ | 1000 ml |

pH adjustment to 7.0 before autoclaving.
Autoclaving 30 min./121°C.

Hereafter a 10 l Chemap A fermenter, filled to 7 l with XYL-10 substrate was incubated with 5% of the fermentation volume. The fermenter was run for 5 days, 30°C, 600 rpm, 1 NL/min. The culture broth is centrifuged for 30 min at 4000 rpm Sorwall RC-3B with a 6000 A rotor, and hereafter filtered through a 10 μm filter. The resulting volume of 7000 ml was then ultrafiltered through a 10,000 MW cut-off membrane using Pellicon ultrafiltration equipment from Millipore. Result: 398 g of liquid.

**EXAMPLE 7**

Oxygen delignified hardwood is treated with a hemicellulase preparation from Malbranchea cinnamomea (produced according to the procedure in Example 1) at pH=8.0, 50°C and a consistency of 10% for 3 hours. An enzyme dosage of 30.0 kEXU/kg is used. After the enzyme treatment the pulp is washed and the kappa numbers determined. A control sample of pulp is treated like the enzyme treated sample but without addition of enzyme.

After the enzyme treatment the pulps are bleached in a C/D-E bleaching sequence using varying amounts of active chlorine in the C/D-stage. The bleaching conditions are as follows:

C/D-stage

| Time: | t = 45 min |
|---|---|
| Temperature: | T = 40°C |
| Consistency: | DS = 10% |
| Substitution: | 30% with $ClO_2$ (as active chlorine) |

E-stage

Time:              t = 1 hr
Temperature:    T = 60°C
Consistency:     DS = 10%
NaOH dosage:     2.0% (w/w) on dry pulp

After the C/D-E stages the kappa number of the pulps are deter-mined.

In Table 1 below the kappa numbers after the enzyme treatment are listed. In Table 2 the kappa numbers after the C/D-E stages are listed together with the amounts of active chlorine used for the bleaching together with the active chlorine multiples.

## Table 1

|  | Kappa number after enzyme treatment |
| --- | --- |
| Control | 11.0 |
| Enzyme | 9.2 |

## Table 2

| Multiple for active Cl in C/D-stage | Kappa number after C/D-E | | Active Cl dosage in C/D | |
| --- | --- | --- | --- | --- |
|  | Control | Enzyme | Control %(w/w) | Enzyme %(w/w) |
| 0.22 | 2.06 | 1.58 | 2.42 | 2.00 |
| 0.18 | 2.71 | 2.00 | 2.00 | 1.67 |
| 0.15 | 3.45 | 2.83 | 1.65 | 1.37 |

It is seen that the multiple for active chlorine can be reduced below the claimed value of 0.2 after the enzyme treatment and at the same time still giving a lower kappa number after C/D-E than for a control bleached with active chlorine in an amount corresponding to a multiple of 0.22.

## EXAMPLE 8

An unbleached hardwood brown stock is treated with a hemicellulase preparation from Bacillus pumilus (produced according to the procedure in Example 2) at the following conditions:

Time:              t = 3 hours
Temperature:    T = 50°C
pH:               pH = 8.0
Consistency:     DS = 10%
Dosage:           715 EXU/kg dry pulp

After the enzyme treatment the pulp is washed with water and bleached in a three stage bleaching sequence, C/D-E-D.

A control is treated in the same way but without addition of enzyme.

Table 3 below shows the kappa numbers of the pulps after the enzyme treatment.

**Table 3**

|  | kappa number | reduction % |
|---|---|---|
| Control | 15.0 | - |
| Bacillus pumilus | 13.6 | 9.2 |

The C/D-E-D bleaching stages are performed under the following conditions:

C/D-stage

Time:          t = 20 min
Temperature:   T = 50°C
Consistency:   DS = 5%
Substitution:  50% with $ClO_2$ (as active chlorine)

E-stage

Time:          t = 1 hr
Temperature:   T = 60°C
Consistency:   DS = 10%
NaOH dosage:   2.0% (w/w) on dry pulp

D-stage

Time:          t = 3 hr
Temperature:   T = 70°C
Consistency    DS = 10%

Both pulps are bleached to a kappa number of 3.5 after the C/D-E stages. For the control 2.8% (w/w) active chlorine is needed in the C/D-stage to reach this kappa number. For the enzyme treated pulp only 2.38% (w/w) active chlorine is needed to reach a kappa number of 3.5. This corresponds to a reduction in active chlorine of 14.5% for the enzyme treated pulp compared to the control in order to reach the desired kappa number.

In the final D-stage both pulps having a kappa number of 3.5 after C/D-E are bleached to their respective brightness ceilings.

For the enzyme treated pulp a chlorine dioxide dosage of 0.99% (w/w) is needed to bring the pulp to a brightness of 87.2% (ISO) brightness. For the control a dosage of 1.2% (w/w) chlorine dioxide is needed to reach a final brightness of 85% (ISO). This shows that the enzyme treatment makes it possible to reduce the dosage of chlorine dioxide in the final D-stage by 17.5% and at the same time elevate the brightness ceiling of the pulp by a 2.2% ISO brightness.

The enzyme treatment of the pulp neither causes strength nor yield loss.

**EXAMPLE 9**

A crude xylanase preparation from Bacillus stearothermophilus (produced according to the procedure in Example 3) was used to pretreat a softwood kraft pulp before a C-E bleaching.

The pulp was treated under the following conditions:

Pulp:              Loblolly Pine kraft brown stock (initial kappa number 24)
pH:                8.5
Time:              1 hour
Temperature:       60°C
Pulp consistency:  3.5%
Dose:              1000 EXU/kg dry pulp

Pulp and enzyme were mixed by hand in plastic bags and kept at constant temperature in a water bath. A control sample was submitted to the same treatment as described above, but without addition of enzyme.

After the enzyme treatment the pulps were bleached under the following conditions:

C-stage

Time:           t = 45 min
Temperature:    T = 40°C
Consistency:    DS = 3.5%

E-stage

Time:           t = 1 hr
Temperature:    T = 70°C
Consistency:    DS = 10%
NaOH dosage:    0.5 times the chlorine charge

In Table 4 the kappa numbers after the C-E stages are listed both for the enzyme treated pulps and for the controls at four different dosages of chlorine in the first stage.

The experiment show that the enzyme treatment at an alkaline pH {8.5} cause a significant drop in kappa number after the C-E bleaching compared to the control.

## Table 4

| Multiple for active chlorine | Kappa number | |
|---|---|---|
| | Enzyme | Control |
| 0.11 | 12.53 | 17.22 |
| 0.14 | 7.50 | 14.16 |
| 0.18 | 6.66 | 10.10 |
| 0.22 | 5.98 | 7.12 |

It is seen that a kappa number of 7.12 (the kappa-number of the control at a multiple of 0.22) can be obtained after an enzyme treatment by means of a multiple of approx. 0.158 (found by linear interpolation between the experimental values). This is a reduction in dosage of chlorine of approx. 30% after the enzyme treatment compared to the control.

### EXAMPLE 10

An unbleached birch kraft pulp was treated with an xylanase rich hemicellulase preparation from Humicola insolens (produced according to the procedure in Example 4). After the enzyme stage the pulps were bleached in a three stage bleaching sequence, C/D-E-D, to a final brightness of 88% ISO.

The pulps were treated under the following conditions:

Enzyme

Time:           t = 3 hours
Temperature:    T = 50°C
Consistency:    DS = 10%
pH:             pH = 9.0
Dosage:         1900 EXU/kg dry pulp

C/D-stage

Time:           t = 45 min
Temperature:    T = 40°C
Consistency:    DS = 5%
Substitution:   30%

### E-stage

| | |
|---|---|
| Time: | t = 1 hr |
| Temperature: | T = 60°C |
| Consistency: | DS = 10% |
| NaOH dosage: | 2% (w/w) on dry pulp |

### D-stage

| | |
|---|---|
| Time: | t = 3 hr |
| Temperature: | T = 70°C |
| Consistency: | DS = 10% |

Both the enzyme treated pulp and the control were bleached to a kappa number of 3.0 after the C/D-E stages. To obtain this kappa number the active chlorine dosage for the enzyme treated pulp can be reduced by 19% down to 2.24% (w/w) compared to the control. The multiple for active chlorine for the enzyme treated pulp is 0.16.

In the final D-stage the control sample reaches the brightness ceiling at 88% (ISO) brightness by means of a dosage of 1.33% w/w $ClO_2$ on dry pulp. The enzyme treated pulp only requires 0.86% (w/w) $ClO_2$ on dry pulp to reach this brightness.

By addition of the reduced amounts of bleaching agents in the two bleaching stages (as active chlorine) it is seen that the treatment of the pulp by means of a hemicellulase preparation from H. insolens makes it possible to reduce the required amount of active chlorine after an enzyme treatment by 28% compared to a control when bleaching the pulp to a final brightness of 88% (ISO).

## EXAMPLE 11

The enzyme preparation from H. insolens that was used for the experiments described in the Example 10 has also been tested on oxygen delignified softwood.

The softwood was treated with the enzyme and afterwards bleached in a C/D-E sequence. The conditions for the enzyme treatment as well as the bleaching were the same as those listed in the above example 10, except for the substitution with chlorine dioxide. The substitution was raised to 50% in this experiment.

After the enzyme treatment the entire amount of the pulp was bleached with active chlorine in an amount corresponding to a multiple of 0.2 in the C/D-stage corresponding to a dosage of 3.36% (w/w) of active chlorine. In Table 5 below the kappa numbers after the C/D-E bleaching are listed for four enzyme treatments with varying dosages of enzyme.

### Table 5

| Enzyme dosage EXU/kg | Kappa number after C/D-E | Reduction in kappa number % |
|---|---|---|
| Control | 3.05 | – |
| 115 | 2.69 | 11.8 |
| 230 | 2.41 | 21.0 |
| 935 | 2.36 | 22.6 |
| 1870 | 2.24 | 26.6 |

It is seen that the enzyme treatment at all four dosages tested reduces the kappa number of the pulp after the C/D-E bleaching rather significantly compared to the control.

When comparing these results with the results presented in Example 7 it is also observed that the hemicellulase preparation shows approximately the same bleach boosting effect on both softwood and hardwood.

## EXAMPLE 12

An unbleached hardwood brown stock pulp was treated with a xylanase containing enzyme preparation

from <u>Thermomonospora fusca</u> (produced according to the procedure in Example 5) and afterwards bleached in a C/D-E bleaching sequence. The conditions for enzyme treatment and bleaching were as follows:

Enzyme

Time:           t = 3 hours
Temperature:    T = 50°C
Consistency:    DS = 10%
pH:             pH = 8.0 and 9.0
Dosage:         200, 400 and 800 EXU/kg of dry pulp

C/D-stage

Time:           t = 45 min
Temperature:    T = 40°C
Consistency:    DS = 5%
Substitution:   50%

E-stage

Time:           t = 1 hr
Temperature:    T = 60°C
Consistency:    DS = 10%
NaOH dosage:    %(w/w) NaOH = 0.5 (%(w/w)Cl$_2$ + %(w/w)ClO$_2$) + 0.3

In the C/D-stage the pulps were bleached using an active chlorine multiple of 0.18. After the E stage the kappa numbers of the pulps were determined. The results are listed in Table 6 below for three enzyme dosages at two pH levels for the enzyme treatment. In Table 6 the reduction in kappa number after an enzyme treatment compared to a control treatment is also listed.

**Table 6**

| Enzyme dosage EXU/kg | Kappa number after C/D-E pH=8.0 | pH=9.0 | Reduction in kappa no pH=8.0 | pH=9.0 |
|---|---|---|---|---|
| Control | 3.75 | 3.89 | – | – |
| 200 | 3.61 | 3.46 | 3.73% | 11.05% |
| 400 | 3.35 | 3.10 | 10.67% | 20.31% |
| 800 | 3.03 | 2.93 | 19.20% | 24.68% |

For this enzyme complex good bleach boosting effects are observed both at pH 8 and 9. The results indicate that the effects obtained at a pH of 9 is better than the effects obtained at a pH of 8.

**EXAMPLE 13**

An unbleached hardwood pulp was treated with a xylanase containing enzyme preparation from <u>Streptomyces olivochromogenes</u> (produced according to the procedure in Example 6) and afterwards bleached in a C/D-E bleaching sequence. The conditions used for both the enzyme treatment and the bleaching are the same as those listed in Example 12.

After the C/D-E bleaching the kappa number of the pulps were determined. The results are listed in Table 7 below. The percentage reduction of the kappa number after an enzyme treatment compared to the control is also listed.

## Table 7

| Enzyme dosage EXU/kg | Kappa number pH=8.0 | pH=9.0 | Reduction in kappa no pH=8.0 | pH=9.0 |
|---|---|---|---|---|
| Control | 3.8 | 3.9 | - | - |
| 80 | 2.8 | 3.2 | 24.3% | 17.0% |
| 160 | 2.5 | 3.0 | 34.1% | 23.4% |
| 320 | 2.5 | 2.7 | 32.5% | 30.3% |

It is observed that good bleach boosting effects are obtained both when the pulp is treated with enzyme at pH 8 and 9.

## EXAMPLE 14

Hardwood kraft pulp was treated with the same enzyme preparation from Streptomyces as used in Example 13 under the same conditions as listed in Example 12. The pulp was treated with enzyme, 160 EXU/kg of dry pulp, at pH 8 and 9. After the enzyme treatment the samples were bleached to the same kappa number after the C/D-E stages.

When bleaching to a kappa number of 3.8 the dosage of active chlorine in the C/D stage can be reduced by 23 and 16 percent compared to a control when enzyme treated at pH 8 and 9, respectively.

Afterwards the pulps were bleached to a final brightness of 89% ISO brightness in a D-stage.

The strength properties of the fully bleached pulps were tested. The strength of the pulps as well as the yield has not been reduced by the enzyme treatment.

## Claims

1. Process for treatment of lignocellulosic chemical pulp comprising a xylanase treatment and a subsequent chlorine treatment, characterized in that the lignocellulosic pulp is treated with an alkaline xylanase at a pH above 7.5, whereafter the thus treated cellulosic pulp is treated with chlorine at an active chlorine multiple of 0.20 or less in the first chlorination stage.

2. Process according to Claim 1, wherein the xylanase treatment is performed at temperatures between 40 and 100°C, preferably between 40 and 80°C, more preferably between 50 and 70°C.

3. Process according to Claim 1 or 2, wherein the xylanase treatment is performed over a period of 15 minutes to 24 hours, preferably between 30 minutes and 5 hours, most preferably between 30 minutes and 3 hours.

4. Process according to Claims 1 - 3, wherein the xylanase treatment takes place at a- consistency of 5-35%, preferably 8-25%, most preferably 8-15%.

5. Process according to Claims 1 - 4, wherein the xylanase is producible by means of the microorganisms Malbranchea cinnamomea, Humicola insolens, Bacillus pumilus, Bacillus stearothermophilus, Thermonmonospora fusca or Streptomyces olivochromogenes.

6. Process according to Claims 1 - 5, wherein an (EOP) treatment is introduced between the xylanase treatment and the chlorine treatment.

7. Process according to Claims 1 - 6, wherein the chlorine multiple is between 0.10 and 0.20.

## Patentansprüche

1. Verfahren zur Behandlung von chemisch vollkommen aufgeschlossener Lignocellulosepulpe, welches eine Xylanase-Behandlung und eine anschließende Chlor-Behandlung umfaßt, dadurch gekennzeichnet, daß die Lignocellulosepulpe mit einer alkalischen Xylanase bei einem pH über 7,5 behandelt wird, woraufhin die so behandelte Cellulosepulpe mit Chlor bei einem Aktivchlor-Vielfachen von 0,20 oder weniger in der ersten Chlorierungsstufe behandelt wird.

2. Verfahren nach Anspruch 1, wobei die Xylanase-Behandlung bei Temperaturen zwischen 40 und 100°C, vorzugsweise zwischen 40 und 80°C, noch bevorzugter zwischen 50 und 70°C durchgeführt wird.

3. Verfahren nach Anspruch 1 oder 2, wobei die Xylanase-Behandlung über einen Zeitraum von 15 Minuten bis 24 Stunden, vorzugsweise zwischen 30 Minuten und 5 Stunden, am bevorzugtesten zwischen 30 Minuten und 3 Stunden durchgeführt wird.

4. Verfahren nach den Ansprüchen 1 - 3, wobei die Xylanase- Behandlung bei einer Konsistenz von 5 - 35%, vorzugsweise 8 - 25%, am bevorzugtesten 8 - 15% stattfindet.

5. Verfahren nach den Ansprüchen 1 - 4, wobei die Xylanase herstellbar ist mit Hilfe der Mikroorganismen Malbranchea cinnamomea, Humicola insolens, Bacillus pumilus, Bacillus stearothermophilus, Thermonmonospora fusca oder Streptomyces olivochromogenes.

6. Verfahren nach den Ansprüchen 1 - 5, wobei eine (EOP)-Behandlung zwischen der Xylanase-Behandlung und der Chlor- Behandlung eingeschoben wird.

7. Verfahren nach den Ansprüchen 1 - 6, wobei das ChlorVielfache zwischen 0,10 und 0,20 liegt.

## Revendications

1. Procédé de traitement de pâte chimique lignocellulosique comprenant un traitement avec une xylanase suivi d'un traitement au chlore, caractérisé en ce que l'on traite la pâte lignocellulosique avec une xylanase alcaline à un pH supérieur à 7,5, et que l'on soumet ensuite la pâte cellulosique ainsi traitée à un traitement au chlore à un multiple de chlore actif inférieur ou égal à 0,20 dans la première étape de chloration.

2. Procédé selon la revendication 1, dans lequel le traitement à la xylanase est effectué à des températures comprises entre 40 et 100°C, de préférence entre 40 et 80°C, de façon plus préférable entre 50 et 70°C.

3. Procédé selon la revendication 1 ou 2, dans lequel la durée du traitement à la xylanase est comprise entre 15 minutes et 24 heures, de préférence entre 30 minutes et 5 heures, de façon la plus préférable entre 30 minutes et 3 heures.

4. Procédé selon les revendications 1 - 3, dans lequel le traitement à la xylanase a lieu à une consistance de 5-35 %, de préférence de 8-25 %, de façon la plus préférable de 8-15 %.

5. Procédé selon les revendications 1 - 4, dans lequel la xylanase peut être produite au moyen des microorganismes Malbrancha cinnamomea, Humicola insolens, Bacillus pumilus, Bacillus stearothermophilus, Thermonmonospora fusca ou Streptomyces olivochromogenes.

6. Procédé selon les revendications 1 - 5, dans lequel on introduit un traitement (EOP) entre le traitement à la xylanase et le traitement au chlore.

7. Procédé selon les revendications 1 - 6, dans lequel le multiple de chlore est compris entre 0,10 et 0,20.